# EUROPEAN PATENT APPLICATION

(11) **EP 2 438 863 A1**
(43) Date of publication of application: **11.04.2012**
(21) Application number: 10186624.2
(22) Date of filing: 05.10.2010
(51) Int. Cl.: A61B 5/16, G10L 17/00

(54) **System and methods for analysis of pause time durations in speech.**

(71) Applicant: Phibio Science AB, 216 13 Limhamn (SE)
(72) Inventor: Erikson, Catarina, 216 13, Limhamn (SE)
(74) Representative: KIPA

(57) **Abstract**

A diagnostic aid system, method, and computer program are disclosed.These are devised for assessment of a brain status of a subject, wherein said brain status comprises a neurodevelopmental disorder or a brain disease induced brain dysfunction, wherein said system is adapted to determine an occurrence and/or stage of said neurodevelopmental order or brain disease induced brain dysfunction in said subject. An apparatus is provided that is adapted to determine said occurrence and/or stage of said neurodevelopmental disorder or brain disease induced brain dysfunction in said subject from speech of said subject, said apparatus comprising units that are operatively connected to each other. The apparatus comprises a unit for registering said speech of said subject over a period of time; a processing unit devised for analyzing said registered speech and configured to determine a plurality of individual pause time components of said registered speech and determine a statistical distribution of said plurality of individual pause time components; and wherein said processing unit is adapted to determine said occurrence and/or stage of said brain disease induced brain dysfunction from said statistical distribution.

## Description

### Field of the Invention

This invention pertains to the field of systems and methods for assessment of an attentional lap (pause time) induced by brain dysfunction which is of developmental origin of the central nervous system (CNS) in children, adolescents and adults, or reflects the aging and disease processes of the CNS in the elderly. More particularly the invention relates to such systems and methods for determining or diagnosing if the person suffers from attentional lapses induced by brain dysfunction or is in risk thereof by analyzing the speech of the person.

### Background of the Invention

A person's ability to concentrate and to focus on external stimuli may be compromised by disturbances in the developmental trajectories of the CNS, by fatigue or intoxication, or by changes in CNS induced by aging processes or age related diseases. During recent years increasing evidence has accumulated that different systems and subsystems in the brain are responsible for such behavioural difficulties. Particular interest has been drawn to the functional correlations and the coherence of activity of widespread neuronal populations which are observed during a resting state (default mode network, DMN) and subsystems of the DMN which are believed to participate during goal-directed activity (task positive network) or not (task negative network). In order to perform goal-directed activity, it is believed that the low frequency coherent neuronal oscillations of the DMN (including the task negative network) are deactivated which allows the activation of task positive networks to perform the task. Thus, the activity of the brain constantly shifts between states of internal tasks such as daydreaming, self-reference, envision of the future, retrieving memories and so on, and functional activation of DMN-opposed (anti correlated) subsystems needed to process and to react to external information, thus neglecting internal tasks. This constant switch between "on" and "off' between different states of the brain may be compromised and such compromise can result in suboptimal performance by the person. One of the behavioural manifestations of such compromise is attentional difficulties in a subject, believed to be due to an imbalance in the strength of neuronal coherence between systems.

Difficulties to concentrate and to be attentive to external information are common in neurodevelopmental disorders of the CNS, such as Attention Deficit Hyperactivity Disorder (ADHD) or Attention Deficit Disorder (ADD). Such difficulties are also seen in subjects suffering from age related diseases, such as dementia, or from various neurological conditions (stroke, tumour, epilepsy).

Attentional fluctuations have commonly been assessed by continuous performance tests such as reaction time tasks. Such tasks are externally driven such that visual or auditory stimuli are presented and the subjects need to react to these stimuli by for example press a button in response to the stimulus. These tasks engage the so called external loop of the brain. This reflexive view of brain function has traditionally been very informative but does not consider the intrinsic capacity of the brain's own ability to switch "on" and "off' between different modes of behaviour, and does not reflect the speed of the so called internal loop of the brain, i.e. the intrinsically generated higher order cognitive processing. A compromise of the self-paced speed of intrinsic brain processing to open-ended external information may in fact be one of the earliest behavioural consequences of brain dysfunction, which can be characterized by moment-to-moment attentional fluctuations.

The brain may be damaged in many various ways by the aging CNS, and CNS-changes may precede clinical evidence of such changes for many decades. This can be seen for example in mild cognitive impairment (MCI), small or large vessel diseases, damage of the blood brain barrier function, atherosclerosis, etc. where clinical symptoms of ongoing brain damaging processes may not be evident until a certain point is reached in the development of such processes.

Diseases, in which such processes are accelerated and clinically evident comprise for instance dementia, Alzheimer's disease (AD); or Multiple sclerosis (MS), but includes also many other CNS-diseases.

A large number of persons are affected by such diseases. Dementia and dementia-associated diseases are actually ranked as the fourth common cause of death in industrialized civilizations of the globe, after cardiac diseases, cancer, and stroke. In Sweden alone, having a population of only nine million, about 150000 - 200.000 persons are suffering from dementia. About 7 percent of the elderly and 20-30 percent of the 85 year old persons suffer from dementia. As the percentage of elderly of the total population will increase due to longer expected life, the absolute number of patients will even increase with time. Therefore, there is a need to identify persons at risk of developing dementia or having a certain degree of dementia as early as possible in order to be able to provide suitable treatment.

Disorders related to the brain also include neurodevelopmental disorders, such as attention deficit hyperactivity disorder (ADHD). The worldwide prevalence of this disorder has been estimated to 3 - 5% in children and adolescents younger than 18 years. Many adults with symptoms of the disorder remain undiagnosed. There is a need to test, facilitate diagnosis, or provide diagnosis of ADHD, and objectively evaluate treatment strategies.

Automated Systems implementing cognitive testing have been disclosed, e.g. in US2006/0194176A1 or EP1205146.

In US2006/0194176A1 a dementia testing apparatus e.g. for senile dementia, is disclosed, which has a test chart that comprises tale including test sentences containing colored words and questions for determining whether words are colored with a color expressed by a colored word. In more detail, an answer obtaining section of the apparatus obtains answers from a patient that are made within predetermined answer time limits to a first and a second examination chart. The first examination chart has inspection sentences where a character group constituting a story including color words each representing color is tinted with plural colors such that individual color word has characters of the same color, requires a determination as to whether the color of characters constituting the color word is the same color as color represented by the color word. The second examination chart has a combination of questions concerning contents of the inspection sentences and answers which are prepared for each question and one of which is to be selected. In a dementia degree inspection section of the apparatus, a dementia degree of the subject based on the answers obtained by the answer obtaining section is determined.

In EP1205146 a patient answer based dementia test system for testing the degree of dementia of a subject is disclosed. The dementia test system which is effective for preventing and finding, at an early stage, an initial sign (initial dementia) of senile dementia. A dementia test apparatus comprising an answer obtaining section for obtaining an answer of a subject to both a dementia degree test chart which requires the subject to exercise a plurality of judgments at the same time and obtain an answer in such a form that correction of judgment is objectively determined, and a dementia factor degree test chart comprising a combination of multiple questions concerning sensibility and a multiplicity of answers alternatively selected from questions prepared for each of the former questions, and a dementia degree test section for testing a dementia degree indicative of the current degree of dementia of the subject based on an answer obtained by the answer obtaining section, and for estimating a future dementia degree of the subject.

However, the test systems of the prior art, such as disclosed in US2006/0194176A1 or EP1205146, suffer from the same drawbacks as manually performed cognitive tests, e.g. a dependency of the test on educational, social and cultural factors, including language, of the subject.

WO 2009/056650 discloses a system for assessment of brain disease induced brain dysfunction based on the accumulated pause time of speech over a period of time. Individual variations in brain function of subjects throughout a period may not be detected, and/or the reliability and/or sensitivity for detecting brain dysfunction may not be sufficient in such method.

Thus, there is a need for a new or improved system and/or method for assessing brain damages caused by brain diseases or a risk for developing such diseases. It is desired that such a system and/or method is providing a reliable diagnosis of brain damage induced brain dysfunctions independent of educational, social and cultural factors, including language, of the subject to be diagnosed.

Hence, an improved system and/or method, e.g. for assessing neurodevelopmental disorders or brain dysfunction caused by diseases of the aging brain or a risk for developing such brain dysfunction, would be advantageous and in particular a system and/or method allowing for increased flexibility, cost-effectiveness, patient comfort and/or independency of educational background and/or cultural factors and language of a subject to be tested, would be advantageous.

### Summary of the Invention

Accordingly, embodiments of the present invention preferably seek to mitigate, alleviate or eliminate one or more deficiencies, disadvantages or issues in the art, such as the above-identified, singly or in any combination by providing a system, a method, a computer program, and a medical workstation according to the appended patent claims.

Random speech is registered and analyzed. Correlations between an accumulated pause time in relation to the total speech time and brain dysfunction are analyzed.

According to a first aspect of the invention, a system is provided, wherein the system is devised for assessment of the brain functional status of a subject, and wherein the brain functional status comprises a neurodevelopmental disorder or a brain disease.The system is adapted to determine an occurrence and/or stage of the neurodevelopmental disorder or brain disease induced brain dysfunction in the subject. The system comprises an apparatus that is adapted to determine the occurrence and/or stage of the neurodevelopmental disorder or brain disease induced brain damage in the subject from speech of the subject. The speech may be random speech of the subject. Alternatively, or in addition, the speech may be based on a naming task, which is arranged and performed independent of the subject's language. The apparatus comprises units that are operatively connected to each other, which comprises a unit for registering the speech of the subject over a period of time; a processing unit devised for analyzing the registered speech and configured to determine a plurality of individual pause time components of the speech and being adapted to determine a statistical distribution of said plurality of individual pause time components; and a processing unit that is adapted to determine the occurrence and/or stage of the brain disease induced brain damage from the statistical distribution.

According to a second aspect of the invention, a method for assessment of a brain status of a subject is provided, wherein the brain status comprises a neurodevelopmental disorder or brain dysfunction induced by a brain disease. The method comprises analyzing speech of the subject and determining aforementioned plurality of individual pause time components of the speech; determining a statistical distribution of said plurality of individual pause time components;
and determining an occurrence and/or stage of the neurodevelopmental disorder or brain dysfunction induced by the brain disease in the subject based on the statistical distribution.

According to a third aspect of the invention, a computer program for processing by a computer is provided. The computer is preferably the one or both of said aforementioned processing units. The computer program is configured for assessment of a brain status of a subject, wherein the brain status comprises a neurodevelopmental disorder or brain dysfunction induced by a brain disease. The computer program comprises a first code segment for analyzing speech of the subject and determining a plurality of individual pause time durations of the speech as defined herein; and a second code segment for determining statistical distribution of said plurality of individual pause time components; and a third code segment for determining an occurrence and/or stage of the brain dysfunction due to a neurodevelopmental disorder or brain dysfunction induced by brain disease in the subject based on this statistical distribution.

Further embodiments of the invention are defined in the dependent claims, wherein features for the second and subsequent aspects of the invention are as for the first aspect mutatis mutandis.

Some embodiments of the invention provide for the following advantages, alone or in any combination, depending on the specific embodiments:
● Measures of processing speed (such as for example using simple colors and shapes, or naming other defined stimuli) are non-invasive, i.e. such tests are easily tolerated by subjects without offending them. In fact, patients are unaware whether they performed good or bad on the test. This is in contrast to knowledge questions raised by for example the MMSE, where patients often become painfully aware of their cognitive problems.
● Embodiments of this invention are implemented in an education and culture-free manner, primarily due to the fact that no knowledge questions are asked. The age-effect is minimal, and lies well within the boundaries of the cut-off limit between normal speed and pathological slowing.
● The assessment of a plurality of individual pause times by embodiments of this invention is the most sensitive measure of information processing speed (which was hitherto not known and wherein a detailed reasoning and example study proving this fact is described in detail further below).
● By assessing a plurality of individual pause times detected throughout a testing period, from a statistical distribution, a more robust, sensitive, reliable method for detecting a neurodevelopmentally induced brain dysfunction or disease induced brain dysfunction is obtained.
● From the primary health care perspective, doctors, nurses or occupational therapists are not offended by using this innovation.
● On the contrary, they are provided with a powerful tool allowing them to rationalize their work and to concentrate on subjects in need of therapeutic care. Based on the assessment results provided by embodiments of the present invention, doctors may easily decide which patients have signs of a decline in processing speed and therefore are at risk for having a neurodevelopmental disorder or developing a brain disease induced brain dysfunction, or not. This information may therefore direct primary health care resources to those patients who are at high risk for having a brain disorder, and who need further assessment for their diagnosis, while saving financial costs for unnecessary evaluations of patients with negative test results.
● A negative test result provided by an embodiment of this invention saves time and worry on behalf of the patient, and is positive information if the patient (or the relative) has e.g. been worried about beginning AD. A negative test result should at the discretion of a doctor, however, be accompanied by a routine clinical evaluation and laboratory screening in order to rule out physical illness.
● Based on experience from individual cases, the speed measure has sometimes been the only measure (including blood tests, MMSE etc.), which has been decisive for further assessment of the patient's complaints of possible brain disease. On the basis of a positive test result solely based on the speed measure, patients have finally received an objective confirmation and a clinical diagnosis (leukoaraios, subclinical white matter infarcts, etc.).
● Some embodiments are cost effective, as e.g. a test session takes a few minutes to perform. This is in practice an essential point as time allocation for each patient in primary health care is short.
● Some embodiments are cost effective and convenient to perform as a handheld apparatus may implement self-testing by said subject.
● The automatized voice recording and analysis of the test results makes the measure objective and independent of an examiner. It works much like a laboratory test.
● Baseline evaluation of test results obtained by some embodiments at a first visit to the doctor may be used as reference values at successive visits. This makes it possible to capture whether progress (cognitive slowing and increased frequency and duration of attentional lapses) has occurred over time. If this is the case and the subject shows a cognitive slowing and /or an increased individual pause time durations at follow-up, this test result forms the basis for further evaluation of the patient, as this slowing of processing speed may suggest a beginning brain degenerative or subcortical brain disorder.

Embodiments of the invention do not comprise cognitive content questions, and thus the above mentioned drawbacks related thereto are avoided. When naming tests are performed in embodiments, these are provided content-independent.

Diseases or conditions to be diagnosed by embodiments of the invention comprise any structural or functional disruption of the brain architecture, including neurodevelopmental disorders or disorders associated with the normal aging process, or associated with any brain disorder of cortical neurodegenerative or brain white matter origin. Furthermore, this includes any induction of inflammatory processes affecting the blood-brain barrier functions of the brain microvascular system, including any genetic risk factors or genetic polymorphisms associated with these processes. Specific diseases associated with mentioned processes, wholly or in part, include: Alzheimer's disease, Multiple sclerosis (MS) or any other sub-cortical white matter disease or demyelinating disease, HIV, malaria, cerebrovascular disease (VaD), encephalitis, traumatic brain injury (TBI), mild cognitive impairment (MCI), fronto-temporal dementia (FTD/FLD), dementia with Lewy body disease (LBD/DLB), and Parkinson's disease (PD).

Conditions such as ADHD may sometimes be difficult to clinically diagnose due to comorbidity of other conditions, but embodiments of the invention can aid in the diagnosis. One of the most evident clinical characteristics of ADHD is frequent laps in the ability to maintain attention over time. Previous research has seldom described the intra-individual moment-to-moment attentional variations which are typical for ADHD. This variability is previously typically described in terms of mean value analysis and different measures for deviations, for example standard deviations. Patients with ADHD often have significantly larger mean values and a larger variation in their performance compared to healthy control individuals of the same age. Such analysis is however not sufficient as such methods do not reveal the individual variations over time (milliseconds and seconds). Another issue with prior methods is that different reaction time tests are used, which essentially measure the ability of the motor systems of the brain to quickly react on an external stimulus, i.e. the so called "outer loop" of the brain function. What is typical for these tests is that a subject should press a button as quickly as possible upon the presentation of a visual or auditory stimuli. In contrast to such tests are those which require a higher degree of mental processing of stimuli, i.e. those tasks which require the activation of the subjects "inner loop" of brain function, i.e. the brains ability to produce its own response in its own pace. This distinction of the brains reflex function in contrast to the self-acting ability of thinking leads to two entirely different aspects of the brains function.

During recent years it has been realized that mean values and deviations are not satisfying statistical measures on the individual variations in individuals having attention deficit disorders such as ADHD. Therefore, studies of time series of any present activity in order to measure variations in the subjects attention over time has become more common. These studies are typically performed for tests involving different reaction tests for measuring the brain's reflex abilities.

Language in the context of the present application is to be understood as a system for expression of thoughts, feelings etc. by use of a burst of spoken sounds. The use of such a system is a distinguishing characteristic of man compared with other animals. Different nations or people use different languages, e.g. French, Chinese, etc. Two or more individuals speaking the same language can communicate with each other via that system. Language in the context of the present application does expressly not include other systematic or nonsystematic means of communicating, such as gestures or animal sounds.

Speech in the context of the present application is to be understood as the act of speaking, i.e. an utterance of the above mentioned spoken words, independent of a language. Speech in the context of the present application does expressly not include the meaning of national or regional language or dialect. Lungs and vocal cords produce basic sounds that result in speech being produced in a manner of articulation determined how tongue, lips, and other speech organs are involved in making a sound make contact. Speech also comprises pause components of silence or absence of sounds, e.g. between words or sentences.

The speed of the brains own ability of thinking (inner loop) may be studied by measuring the length of the pause times that are occurring when a subject is required to name a series of simple, colored figures. This has been described in patent application PCT/SE2010/050480, and published patent application WO 2009/056650, which are incorporated herein by reference in their entirety for all purposes. In the aforementioned patent applications the total pause time has been analyzed, and it has been shown that the longer the total pause time is for completing the task, the slower the process of thinking required for completing the task. This is true for both young people with ADHD and for patients with Alzheimer's decease. A slow process of thinking may be due to difficulties for the individual to maintain attention, and variations in the attention of an individual are common symptoms at ADHD. Difficulties to maintain attention is also occurring at Alzheimer's decease. A variation in the attention is defined as a pause time (time of hesitation) that exceeds a normal threshold value (+/- a standard deviation).

Aforementioned WO 2009/056650 discloses a system for assessment of brain disease induced brain dysfunction based on the accumulated pause time of speech over a period of time. Individual variations in brain function of subjects throughout a period may not be detected, e.g. as pause time frequency is discarded, and/or the reliability and/or sensitivity for detecting brain dysfunction may not be sufficient in such method.

Aforementioned patent application PCT/SE2010/050480 by the applicant have described an improved pause time (shorter pause time) after 1 h treatment of a subject with ADHD with stimulant medication (methylphenidate).

Determination of a statistical distribution of a plurality of individual pause time components has not previously been disclosed. This has the advantage that the individual variations may be resolved, throughout the testing period, which has previously been unresolved in the mean value analysis. The occurrence and/or stage of said brain disease induced brain dysfunction from the statistical distribution, such as a chronological distribution of the individual pause times during a test, may accordingly be determined. Information previously discarded, e.g. when the pause times occur, and how their length and occurrence (frequency) varies over time, may now be used to characterize the brain function in more detail. The reliability is improved as abnormal deviations throughout the test period may be resolved and investigated. Measuring errors may thus also be detected. Further, even a tendency of a deficiency in a subject's brain function may be detected, at an earlier stage, as the peaks of the individual pause times are resolved in the pause time spectra, in contrast to a previous situation where this information was discarded in the accumulated pause time. The threshold for detecting a change in brain function is thereby lowered, i.e. the sensitivity of the method is increased, and suitable treatment may be introduced at an earlier stage.

The parameters in the pause time spectra such as the individual length and occurrence (frequency) of the pauses may advantageously be used for determining the effects of medical treatment of ADHD or dementia. The effect of such treatment on these parameters is previously unknown. Moreover, these parameters may be used to differentiate patients with ADHD and other neurodevelopmental disabilities, e.g. global learning disability or mental retardation, or neurological disorders such as epilepsy, brain tumors etc. The effect of such conditions on the aforementioned parameters is previously unknown.

A reliable diagnosis of brain dysfunction of neurodevelopmental origin or induced by brain disease has hitherto not been feasible with systems from the prior art, which is a major drawback, at least with regard to flexibility of the systems for use with different subjects, as mentioned above in the background section.

In embodiments the total length of speech is not predefined. This provides for a patient-convenient testing environment without stress. There is no pre-defined time limit for a certain naming task for which the plurality of individual pause times are determined, for assessing a statistical distribution of the plurality of individual pause times. Rather the task is fixed, but not the time for the task. All voice information from an entire measurement period is made use of. Measurement time starts e.g. when stimuli are presented and stopped when the subject so indicates, e.g. by pressing a stop button when a naming task is finished or the subject aborts the speech registration of other reasons (e.g. tired).

It should be emphasized that the term "comprises/comprising" when used in this specification is taken to specify the presence of stated features, integers, steps or components but does not preclude the presence or addition of one or more other features, integers, steps, components or groups thereof.

### Brief Description of the Drawings

These and other aspects, features and advantages of which embodiments of the invention are capable of will be apparent and elucidated from the following description of embodiments of the present invention, reference being made to the accompanying drawings, in which
Fig. 1 is a flow chart illustrating an embodiment of a method;
Fig. 2 is a schematic illustration of an embodiment of an apparatus;
Fig. 3 is a graph showing an excerpt from a registered speech signal of a subject;
Fig. 4 is a color and naming chart for a color and form naming sequence test; and
Figs. 5a-b show pause time distribution with the pause time on the y-axis (milliseconds), and a plurality of individual pauses on the x-axis in chronological order. A 17-year old female patient with ADHD has been tested in an non-medicated state (a) and after a treatment with methylphenidate for 1 h (b).
Fig. 6 shows a pause time distribution of a 12 year-old male patient with ADHD (open circles) compared to healthy young subjects (solid triangles).
Fig. 7 shows a pause time distribution for a 10 year-old patient with diagnosed epilepsy (open circles), compared to healthy subjects in the same age (solid triangles).
Fig. 8 shows a pause time distribution for a 81 year-old female patient with diagnosed Alzheimer's decease (open circles) compared to healthy control subjects around the same age (solid triangles). Vertical lines (solid triangles) indicates one standard deviation.
Fig. 9 shows a pause time distribution for a 74 year-old male patient with diagnosed Alzheimer's decease (open circles) compared to healthy control subjects (solid triangles,+/- one standard deviation).
Fig. 10 shows a pause time distribution of healthy young subjects (solid triangles), young patients with diagnosed ADHD (solid circles in the middle of the figure), and young patients with other diagnosed neurological disabilities (solid circles in the upper part of the figure).
Fig. 11 is a schematic illustration of a method according to an embodiment of the invention, for identifying a stimulus recognition time, a memory retrieval time and pause time for a stimulus.

### Detailed description of embodiments

Specific embodiments of the invention will now be described with reference to the accompanying drawings. This invention may, however, be embodied in many different forms and should not be construed as limited to the embodiments set forth herein; rather, these embodiments are provided so that this disclosure will be thorough and complete, and will fully convey the scope of the invention to those skilled in the art. The terminology used in the detailed description of the embodiments illustrated in the accompanying drawings is not intended to be limiting of the invention. In the drawings, like numbers refer to like elements.

### Method

A method for assessment of a functional brain status of a subject is now described; wherein the functional brain status comprises a neurodevelopmental disorder or a brain disease. The method comprises analyzing speech of the subject and determining a plurality of individual pause components of the speech; determining a statistical distribution of the plurality of individual pause time components; and determining an occurrence and/or stage of the neurodevelopmental disorder or the brain disease in the subject based on the statistical distribution. The pause component, absence of sound, is a key component of the present invention. By determining the total (accumulated) pause time duration of a total duration of speech, adding together all recorded individual pause components during a registration, facilitates an information carrier that is more easily investigated than previously.

In more detail, the method 100 comprises a number of steps 101-104.
101: An untimed training session is performed, whereby the subject is accustomed to the name of different colors and different shapes, and their combinations. In contrast to the unequivocal names of the colors, the subject defines the names of the shapes on her/his own. This procedure is used to minimize the influence of memory and allow for high automaticity in the naming fluency. In other embodiments, different parameters and/or numbers thereof may be used instead of colors and shapes. The stimuli may be shown on a screen.
102: A plate with different shapes (e.g. 40 exemplars, but not limited to this number) is presented to the subject. The subject is asked to name the stimuli as quickly as possible, row by row to the end of the plate. The color is named first, then the shape. The voice recording starts when the subject presses a start button and begins to name the stimuli, and ends when the subject presses a stop button after said subject has named the last stimulus on that particular plate. By using a computer and a digital random re-ordering of the stimulus order and the thus obtained random sequence of color and shape combinations occurs each time a suitable stimuli presenting program is started by said subject. This procedure eliminates any effects of learning and memory of the order of presentations of the stimuli or their combinations.
103. The voice recordings are stored in the memory of an embodiment of an apparatus of the present system, e.g. a handheld recording device. Individual pauses and articulation compartments of the voice recordings are automatically analyzed. A plurality of pause times of any length (milliseconds) is assessed and measured for each particular and randomly generated stimulus set. Pause times shorter than 100 ms may be discarded.

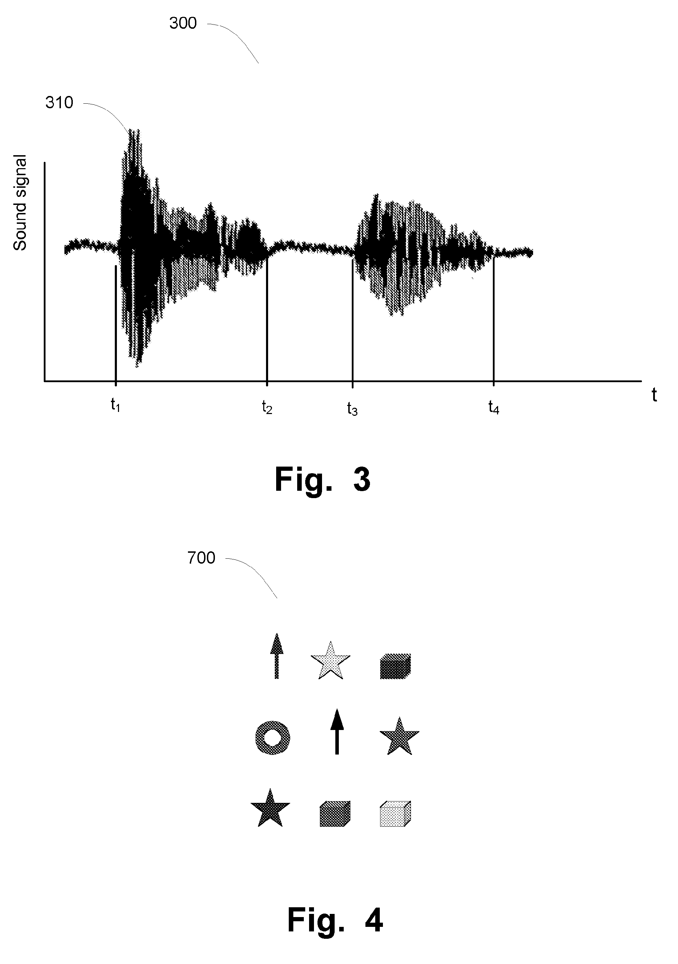

A statistical distribution of the individual pause times, such as a chronological distribution, is determined for the stimulus set. The distribution may be imposed on a range of normal reference or threshold values for a diagnosis. A threshold value may be defined as a number of individual pause times exceeding a threshold duration for a pause time. Non-overlapping pause times of the statistical distribution compared to a range of reference values may be detected for immediate indications of abnormal deviations in a temporally resolved manner. The pause time durations are obtained between all of the vocal bursts recorded during the overt naming of a randomly generated order and a random order of any number of combinations of different colors and different shapes, e.g. four colors and four shapes. One example of a randomly generated set of stimuli 700 is presented in Fig. 4, and one excerpt of a recorded time series showing examples of pause durations, is shown in Fig. 3.

Instead of a naming task, random speech may be registered and analyzed in other embodiments.

The durations (milliseconds) and the statistical distribution of individual pause times are analyzed for a diagnosis of presence or absence of brain dysfunction.

Naming errors are not automatically recorded. The reason for this is that naming errors (misnaming of the stimulus, change of naming order) not significantly contribute to the total naming time. In embodiments pause time includes the inter-word pause times between vocal bursts of overt articulation.

Information processing speed (i.e. mental speed) is measured by several tests, but the definition of what is actually measured by these test instruments varies. This means that one and the same tests (for example Digit Symbol, or Stroop Color-Word test, Trail making test, etc.) is interpreted as measuring mental speed in one study, while in other studies the same test is assumed to measure mental flexibility. This is a frequently occurring issue of definition and face validity of test instruments. Reaction time is often used as a measure of psychomotor speed or processing speed. As will be explained in more detail below, there are several different measures of processing speed, comprising decision speed, perceptual speed, psychomotor speed, reaction time, and psychophysical speed. These different components are included in the "pause time" (i.e. preparation and information processing) that is measured, in addition to attentional lapses. Empirical evidence shows that articulation and pause time are two separate components of the mental processes subserving serial naming tasks, and these two components are not correlated with each other when a verbal response is measured.

### System and Apparatus

In order to perform the test, some embodiments of an apparatus to perform the above describe method comprise a computer having one or more processing units, a microphone, and a speech analysis system. These components may be incorporated into a hand-held computer device which is easy to use, and which calculates the duration of individual pauses as they occur in chronological order of presentation. Alternatively, a medical workstation may be used for performing the test.

Analyzed parameters may be automatically compared with age-matched normal ranges of reference values.

Such an apparatus is provided in a system that is devised for assessment of a brain status of a subject, and wherein the brain status comprises a neurodevelopmental disorder or the risk for and/or the presence of a brain disease induced brain dysfunction. The system is adapted to determine an occurrence and/or stage of the brain dysfunction of neurodevelopmental origin or induced by the brain disease in the subject. The system comprises an apparatus that is adapted to determine the occurrence and/or presence of the neurodevelopmental disorder or brain dysfunction induced by the brain disease in the subject from the plurality of individual pause durations between speech sounds produced by the subject. The apparatus comprises units that are operatively connected to each other, which comprises a unit for registering the speech of the subject over a period of time; a processing unit devised for analyzing the registered speech and configured to determine a plurality of individual pause component of the speech and determine a statistical distribution of said plurality of individual pause time components; and a processing unit that is adapted to determine the occurrence and/or stage of the brain dysfunction of neurodevelopmental origin or induced by the brain disease from the statistical distribution.

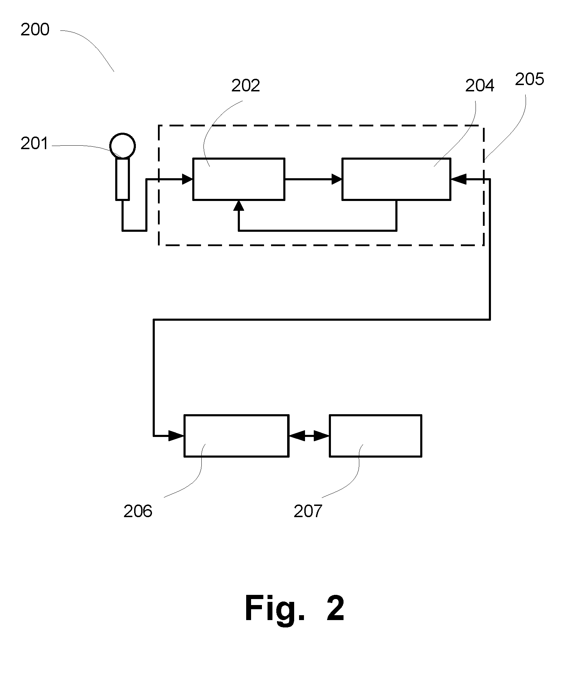

In more detail, Fig. 2 is a schematic illustration of an embodiment of such an apparatus 200 and Fig. 3 is a graph 300 showing an excerpt from a registered speech signal 310 of a subject, with several pauses between vocal bursts.

Apparatus 200 comprises a microphone 201 for registering speech of a subject. The microphone 201 may be any known microphone suitable for registering voice signals and converting these to electrical signals for further processing in the apparatus 200. The microphone 201 is compatible with subsequent processing units, such as Digital Signal Processing (DSP) units, Analog Digital (A/D) converters, processing units, etc. Unit 202 may digitize the signal from the microphone 201 and/or apply a gain control. The converted and/or adjusted signal is then provided to a processing unit 204, which may be a control and sound processing unit.

Units 202 and 204 may be provided as a DSP subsystem that is commercially available. The DSP system communicates with an analyzing unit 206. DSP system 206 may also comprise a memory 207 for, at least temporary, storing or recording the registered speech. The analyzing unit 206 may determine a plurality of individual pause time components of the speech, e.g. from a stored speech signal. An example is given in Fig. 3, where a sound signal 310, corresponding to the vocal bursts of speech of a subject, comprises two exemplary words uttered by the subject between times t1 and t2, as well as between times t3 and t4. A pause time is given between times t2 and t3.

The analyzing unit 206 may determine a statistical distribution of the individual pause times from the speech. Another unit, or analyzing unit 206, is adapted to determine the occurrence and/or stage of said neurodevelopmental disorder or brain disease induced brain dysfunction from the statistical distribution of individual pause times. The distribution may accordingly be indicative of any abnormal pause time values associated with any event during recording of the speech signal. Abnormalities or indications of deviations may be automatically detected by analyzing unit 206. Abnormalities may also include deficiencies in the equipment used for registering the speech data, and/or any brain function, thereby increasing the reliability of the system. The distribution of individual pause times allows for continuous monitoring or subsequent follow-up of any isolated or reoccurring abnormalities. The statistical distribution may be a chronological distribution of the plurality of individual pause time components.

The unit 206 may compare the plurality of individual pause time components of the registered speech with pause time component threshold values to determine the occurrence and/or stage of the neurodevelopmental disorder or brain disease induced brain dysfunction. The pause time component threshold values may comprise different criteria that may be input to the system. The threshold values may comprise a predefined number of the plurality of individual pause times exceeding a pause time threshold or limit duration. For example, if the number of individual pause times exceeding a duration of 1000 ms is amounting to 3 individual pause times, the unit 206 may determine that a abnormal deviation has occurred, which may be indicative of brain dysfunction. Further, an increase or decrease of abnormal deviations may be indicative of a medication. The pause time threshold duration is may be predefined threshold duration outside of +/- one standard deviation of a reference pause time of a healthy subject.

The reference pause time may be a range of reference pause times of a healthy population, and the unit 206 may compare the values of pause times in the reference range with the statistical distribution of individual pause times for determining overlapping or non-overlapping values for determining the occurrence and/or stage of said neurodevelopmental disorder or brain disease induced brain dysfunction. The range of normal reference or threshold values stored in memory 207 for a diagnosis. Hence, non-overlapping pause times of the statistical distribution compared to a range of reference values may be detected for immediate indications of abnormal deviations in a temporally resolved manner.

The apparatus 200 further comprises a human user interface for showing the results of the test and communicating with the user.

Some of the embodiments of the present invention may constitute a hand-held device. The hand-held device may in addition comprise an internal microphone or capability for a microphone which may be connected by wire or wire-less, e.g. by Blue Tooth, IR or any other transmission means.

Some of the embodiments may be a software implementation to be executed on a workstation, e.g. computer, laptop. Moreover, some embodiments may additionally comprise a hardware integrated chip with the system integrated to be connected to the workstation, computer or laptop.

The analyzing unit 206 may be comprised in other processing units of the apparatus. Likewise memory 207 may be part of other memory units of the apparatus.

Further embodiments may comprise a USB dongle, (Universal serial bus), to be connected to the workstation, computer or laptop from which the system is executed as a software code or to unlock the system.

Fig. 4 is an illustration of some examples of color and shape combinations, i.e. sets of stimuli, of a cognitive test for the assessment in the method. A stimuli may comprise a sequence of individual visual and/or auditive stimuli in time for naming by the subject. Alternatively or in combination, a full set of stimuli may be presented to the subject at one time, whereupon the subject should name the full set as quickly as possible. In the example, a visual set of stimuli is shown, comprising four different shapes. The shapes may have any of four different colors (e.g. black, red, yellow, blue). The method uses either a larger predefined set of such combinations or an undefined set of such combinations, all of which may be randomly generated and randomly ordered by the method. Such a chart may be provided virtually via a user interface, e.g. of a medical workstation, to the subject to be tested.

Some embodiments of the invention are implemented in a medical workstation. The medical workstation comprises the usual computer components like a central processing unit (CPU), memory, interfaces, etc. Moreover, it is equipped with appropriate software for processing sound data received from sound data input sources, such as data obtained from microphone devices.

A computer program for processing by a computer is provided is some embodiments. The computer program is configured for assessment of a brain status of a subject, wherein the brain status comprises a neurodevelopmental disorder or a brain dysfunction induced by a brain disease. The computer program comprises a first code segment for analyzing speech of the subject and determining a plurality of individual pause component of the speech; a second code segment for determining a statistical distribution of said plurality of individual pause time components; and a third code segment for determining an occurrence and/or stage of the brain damage induced by brain disease in the subject based on the statistical distribution.

The computer program may for instance be stored on a computer readable medium, accessible by the medical workstation.

The medical workstation may further comprise a monitor, for instance for the display of rendered visualizations, as well as suitable human interface devices, like a keyboard, mouse, etc., e.g. for interacting with the medical workstation. The medical workstation may be part of a system. The medical workstation may also provide data for suggesting treatments based on the assessment outcome. The medical workstation may have a graphical user interface for computer-based assessment of brain damage induces brain dysfunctions. The graphical user interface may comprise components for visualizing the methods described above in this specification or recited in the attached claims.

Embodiments of the system or apparatus described herein may advantageously be implemented and used for carrying out a method, such as the above described or the following method.

A method for assessment of a brain status of a subject, wherein the brain status comprises a neurodevelopmental disorder or a brain disease induced brain dysfunction, wherein the method comprises analyzing speech of the subject and determining a plurality of individual pause components of the speech, as defined herein; determining a statistical distribution of said plurality of individual pause time components; and determining an occurrence and/or stage of the neurodevelopmental disorder or brain disease induced brain dysfunction in the subject based on the statistical distribution.

The method may comprise registering the speech and/or recording the speech of the subject over a period of time; and wherein the analysis of the speech comprises the analysis of the registered speech and/or the recorded speech for determining the length of the pause component between vocal bursts of the speech.

In the method the analyzing the overt speech of the subject may be performed irrespective of a language of the speech.

In the method the assessment may be a cognitive test based assessment, comprising the subject freely defining parameters of the cognitive test.

The method may comprise providing a basis for medical personal for deciding if a subject has signs of a neurodevelopmental disorder or a brain disease induced brain dysfunction or not.

The method may comprise directing primary health care resources to those subjects who are at high risk for having a brain disease induced brain dysfunction, and who need further assessment for their diagnosis, while saving financial costs for unnecessary evaluations of patients with negative test results.

The method may comprise basing the occurrence and/or stage of the neurodevelopmental disorder or the brain disease induced brain dysfunction on a threshold value of the individual pause time components.

The method may further comprise determining the occurrence and/or stage of the neurodevelopmental disorder or the brain disease induced brain dysfunction by comparing the duration of the individual pause times with normal reference values.

The method wherein the determining of the occurrence and/or stage of the neurodevelopmental disorder or the brain disease induced brain dysfunction from the individual pause components may comprise associating an increase in individual pause times with white matter function/dysfunction and/or cerebrovascular dysfunction, in either healthy aging, mild cognitive impairment (MCI) or dementia.

In the method the assessment may be cognitive test based assessment, wherein the subject is free to define parameters of the cognitive test, wherein the cognitive test provides measures of processing speed, such as for example using simple colors and shapes, or naming other defined stimuli, and is non-invasive.

The method wherein the cognitive test may be implemented in an education and culture-free manner, and wherein the cognitive test does not comprise questions related to knowledge of the subject.

In the method the brain disease induced brain dysfunction may be of developmental origin of the central nervous system (CNS), or reflect the aging and disease processes of the CNS in the elderly.

In embodiments of the method the neurodelvelopmental disorders may be related to ADHD, autism spectrum disorders, global learning deficits or mental retardation, or comorbid disorders of neurological origin (e.g. brain tumour, epilepsy etc.) while brain disease induced brain dysfunction may be related to dementia, such as Alzheimer's disease; Multiple sclerosis (MS); Parkinson's disease (PD); dementia with Lewy bodies (DLB/LDB); Amytrophic Lateral Sclerosis (ALS); subcortical white matter disease or demyelinating disease; HIV; malaria; cerebrovascular disease (VaD); encephalitis; traumatic brain injury (TBI); mild cognitive impairment (MCI); traumatic brain injury (TBI); effects of street drugs; alcohol abuse; side effects of prescribed drugs and/or pharmaceutical drug treatments; and diseases of other bodily organs such as heart, liver, lung or otherwise.

Also, the system or apparatus may be used for assessing the status of brain disease induced brain dysfunction in a subject, wherein the brain disease induced brain dysfunction is related to dementia, such as Alzheimer's disease; Multiple sclerosis (MS); Parkinson's disease (PD); dementia with Lewy bodies (DLB/LDB); Amytrophic Lateral Sclerosis (ALS); subcortical white matter disease or demyelinating disease; HIV; malaria; cerebrovascular disease (VaD); encephalitis; traumatic brain injury (TBI); mild cognitive impairment (MCI); traumatic brain injury (TBI); effects of street drugs; alcohol abuse; or side effects of prescribed drugs and/or pharmaceutical drug treatments, and diseases of other bodily organs such as heart, liver, lung or otherwise.

The above described computer program may in some embodiments enable carrying out embodiments of the above described method.

### Results

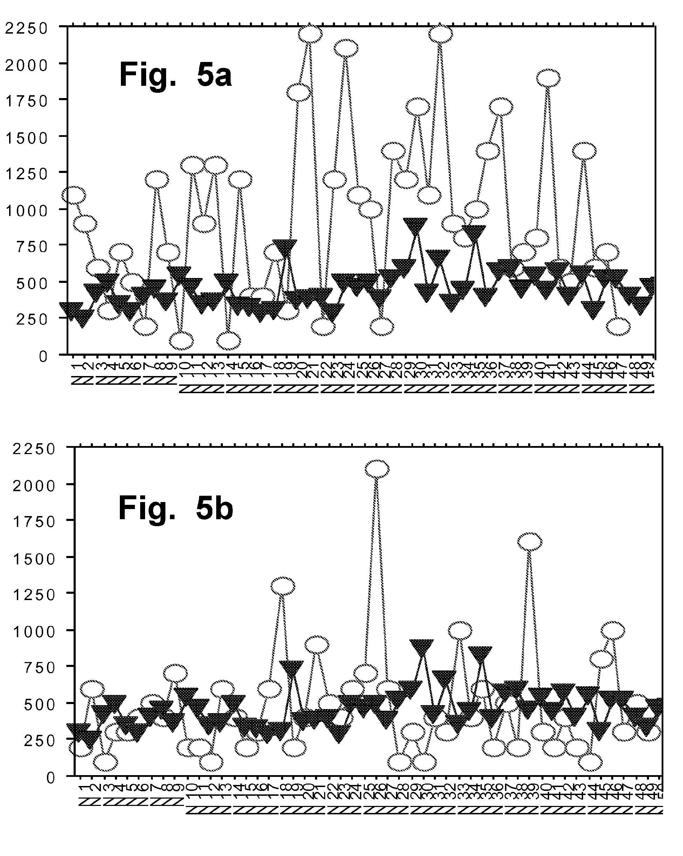

Figs. 5a-b show pause time distributions with the pause time on the y-axis (milliseconds), and the individual pauses on the x-axis in chronological order, starting with a first pause time denoted N1. A 17-year old female patient with ADHD has been tested in an non-medicated state (a) and after a treatment with methylphenidate for 1 h (b). Healthy subjects are displayed as solid triangles, and the patient as open circles. The deviation of the distribution of the individual pause times from the range of reference values (solid triangles) are indicative of the extent of the brain dysfunction. From the pause time distribution it is clear that the frequency and length of the pause times are significantly reduced after the medical treatment. The distribution of pause times may indicate critical deviations associated with isolated events.

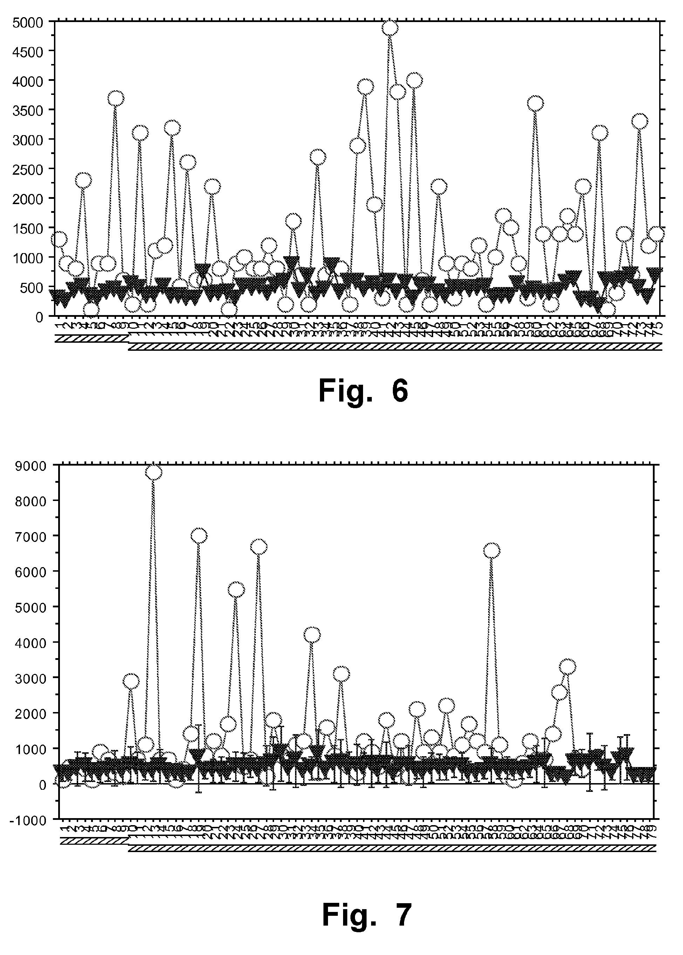

Fig. 6 shows a pause time distribution of a 12 year-old male patient with ADHD and also recent heart surgery. The test is made in connection with medication. A high frequency of pause times with long durations are shown for the patient (open circles) compared to healthy young subjects (solid triangles).

Fig. 7 shows a pause time distribution for a 10 year-old patient with diagnosed epilepsy (open circles), compared to healthy subjects in the same age (solid triangles).

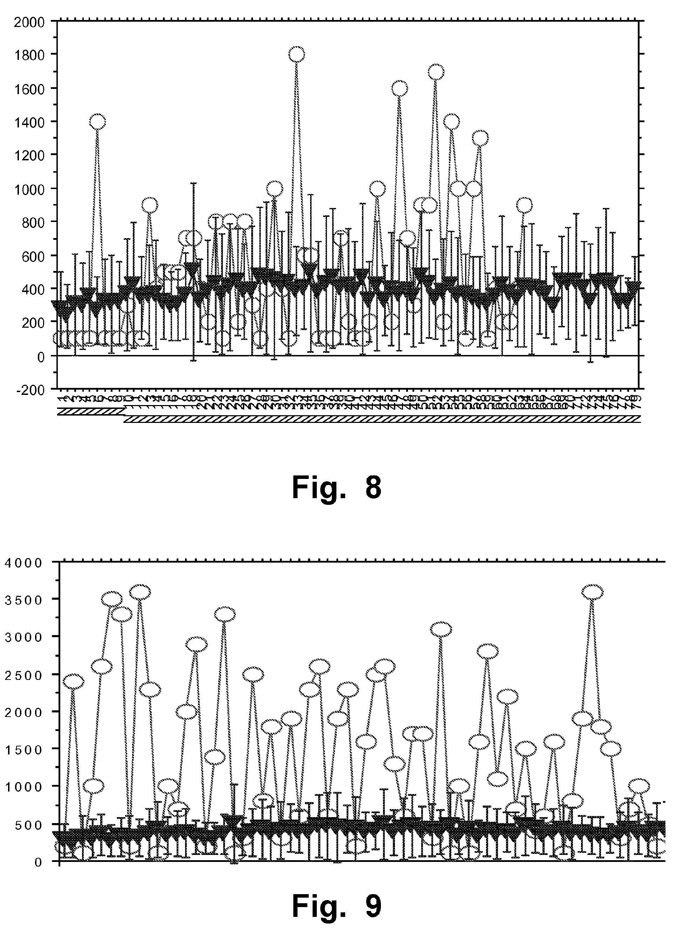

Fig. 8 shows a pause time distribution for a 81 year-old female patient with diagnosed Alzheimer's decease. The duration of the test was 64.8 seconds in order for the patient to complete the test. During this time a moderate frequency of long pause times were detected (open circles) compared to healthy control subjects around the same age (solid triangles) Vertical lines (solid triangles) indicates one standard deviation. In this case, a predefined number of individual pause times exceeding a pause time threshold duration may be set. For example, a predefined threshold duration of 1100 ms may be set, which is outside the standard deviation of the reference range (solid triangles). The number of individual pause times exceeding the pause time threshold duration is in Fig. 8 amounting to 6 individual pause times. Accordingly, any threshold below this number of individual pause times would be indicative of a brain dysfunction.

The individual pause times not overlapping with the range of reference values, e.g. individual pause times outside +/- one standard deviation, may be determined throughout the temporally resolved pause time distribution, to determine the occurrence and/or stage of the brain disease induced brain dysfunction. An array or index may be recorded indicating the occurrence of such deviations in a temporally resolved manner, which may be indicative of certain critical events or parts of a cognitive test performed by a subject, thereby taking into account any variations in the range of reference values determined for the corresponding test.

A compromise of the self-paced speed of intrinsic brain processing to open-ended external information may in fact be one of the earliest behavioural consequences of brain dysfunction, which can be characterized by
Excessive pause time durations of low frequencies (0.01 - 0.1 Hz) may be evidence for moment-to-moment attentional fluctuations seen for example in Alzheimer's disease. This is a characteristic of the compromise of the self-paced speed of intrinsic brain processing to open-ended external information, which may in fact be one of the earliest behavioural consequences of brain dysfunction, as discussed previously.

Fig. 9 shows a pause time distribution for a 74 year-old male patient with diagnosed Alzheimer's decease with a high frequency of long pause times (open circles) compared to healthy control subjects(solid triangles,+/- one standard deviation). From the pause time spectra it is seen that there are relatively small variations in the lengths of the pause time during the test of 138.1 seconds.

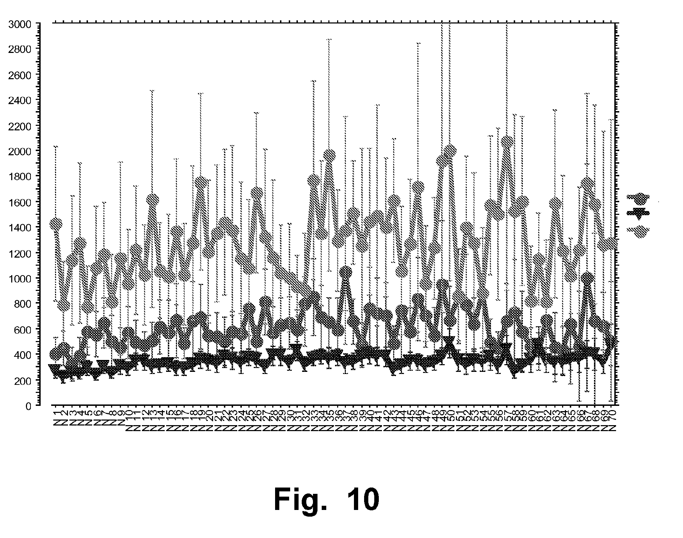

Fig. 10 shows a pause time distribution of healthy young subjects (solid triangles), young patients with diagnosed ADHD (solid circles in the middle of the figure), and young patients with other diagnosed neurological disabilities (solid circles in the upper part of the figure). Vertical lines within each group indicates +/- one standard deviation. It is seen that the variation differs significantly between the groups of patients, and is largest for the group of patients with other disabilities than ADHD.

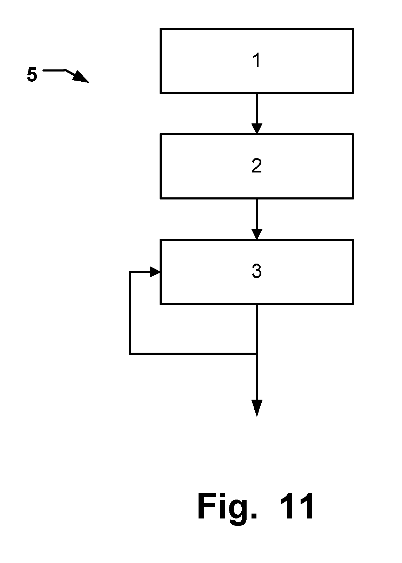

Fig. 11 is a schematic illustration of an embodiment of the invention, showing a method 5, comprising the below steps 1 to 3. This embodiments make use of the assessment of a recognition time and memory retrieval time and their respective contribution to a pause time duration.

### Step 1: Assessment is made of recognition time for a stimulus - which is a part of the pause time measured in step 3).

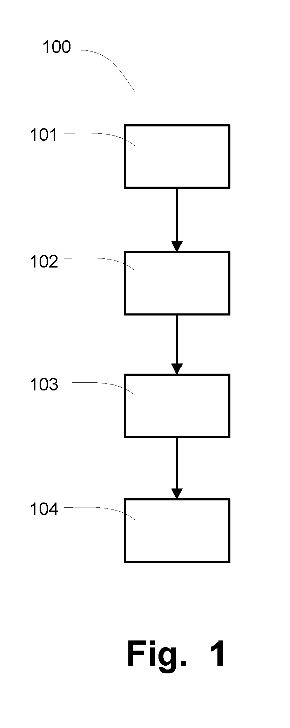

Identification of the stimulus recognition time is performed (Fig.1 in PCT/SE2010/050480). A stimulus S is shown for a stimulation time ts (in parts of a second in Fig. 1). The stimulus S may be shown repeatedly with increasing time until a threshold time is reliably determined where the subject correctly identifies the presented stimulus. Adjustment is made of the lowest time level to correctly recognise the stimuli. For instance, the adjustment starts at the fastest level (0.1 seconds) and this time of stimulus presentation ts is increased successively until a stable recognition is achieved. Step 3 uses this recognition time threshold ts as a default time, as described below.

Thus, it is probed at which stimulus S presentation time the subject is able to correctly identify and name the stimulus. This is usually performed or supervised by an examiner, but may also be made software based by the subject alone using a suitable programmed apparatus. The stimulus presentation time can be changed at discrete levels ranging from the lowest level of 0.1 seconds up to the highest level of 1.0 second. The examiner establishes the lowest level for correct identification of the stimulus through the correct verbal response by the subject. When this presentation time is identified, the examiner (or subject) presses the OK-button 49 on the apparatus 4 (see Fig. 4 in PCT/SE2010/050480). This OK-signal is then automatically used as the presentation time of the stimulus in Step 3. In this manner a reliable and correct identification of the stimuli is ensured at a minimum presentation time. Thereby the component of recognition time for a stimulus - which is a part of the pause time - is reliably determinable.

### Step 2: Assessment is made of a memory retrieval time tr for a stimulus or probe of working memory - which is a part of the pause time measured in step 3.

A sequence or series 21 of a first number of stimuli , e.g. four colour stimuli, S1. S2, S3, S4 (see Fig. 2 in PCT/SE2010/050480), which are e.g. used as default stimuli, may be presented on a screen of an apparatus (see Fig. 4 in PCT/SE2010/050480), over time t. Each first stimuli is visually presented at the set presentation time ts defined in Step 1. The presentation of the stimuli is made in successive order with pre-determined intervals, such as one second intervals, until all stimuli have been shown. The subject is instructed to name the series of first stimuli, in the embodiment the four colour stimuli S1-S4 correctly and in the correct order as soon as all four stimuli have been presented.

At offset of the last stimulus - here S4 - sound recording SR of the anticipated speech is started and the pause time until speech actually starts after starting the SR is automatically analysed. Retrieval time tr for each individual stimulus is calculated as the mean pause time, i.e. the entire mean pause time divided by the number of presented stimuli - in the example for all four stimuli divided by four. The value for tr-mean is automatically stored, and is used by the apparatus 4 (Fig. 4 in PCT/SE2010/050480) or method to calculate the amount of retrieval time contributing to the individual pause times occurring in Step 3.

This procedure may be repeated for a second series of stimuli S5, S6, S7, S8, e.g. four shapes that are different than the shapes of the first series.

This step 2 is a test of short-term memory and a measurement value therefor is provided by tr_mean. In more detail, by dividing the total pause time by the number of stimuli, the memory retrieval time tr_mean for one stimulus is determined.

In other embodiments, a different number of stimuli may be used.

### Step 3:

The method comprises repetitively in a sequence present on a display a pre-defined number of different stimuli individually and in an order, each stimuli being presented during a presentation time, and with a presentation pause of pre-defined length in-between the individual presentations. The presentation time may be the pre-defined recognition time determined previously. After presenting of the last stimuli is finished, for every repetition in the sequence, a plurality of individual pause times is measured, wherein each individual pause time is a pause time until articulation of the subject in each repetition of the sequence starts in the sound signal (or pre-registered speech) for naming the presented stimuli as quickly as possible and in said order. A statistical distribution of the plurality of individual pause times is determined. The occurrence and/or stage of a brain disease induced brain dysfunction is determined from said the statistical distribution according to the discussion above. An apparatus 4 (Fig. 4 in PCT/SE2010/050480) with control unit 720 (Fig. 6 in PCT/SE2010/050480) may be adapted to perform the method in step 3, and also the method in step 1 and 2.

Step 3 may be preceded by determination of a recognition time for a stimulus as a first pause time component, and the determination of a memory retrieval time for a stimulus as a second pause time component, as discussed under steps 1 and 2 above.

The stimuli may be presented pair wise. The method is described in more detail below.

The stimuli are discrete and the presentation time is separated by a pause time interval (presentation pause time). The minimum time (ts) to correctly identify the stimuli is determined by step 1. The presentation time is preferably as large as ts, in order to ensure a reliable recognition of the two random stimuli S1 and S2 by the subject.

The presentation pause time is adjustable and e.g. in the range of 0.1 seconds. This time interval of presentation pause can be adjusted, but a default value of e.g. 0,1 seconds is used. The two stimuli S1, S2 (e.g. colour and shape) are separated by 0.1 second as a default value, thereby creating two discrete stimuli. This default value is a dynamic feature of the method and may be increased, e.g. for specifically probing a memory component by the present testing.

The individual stimuli S1, S2 as well as the order of a pair wise presentation of the stimuli S1, S2 may be randomised (i.e. in the example sometimes a colour appears as the first stimulus S1, sometimes a shape appears first). The subject is required to always name the stimulus presentation in the correct order. Thus, the first presented stimulus S1 is to be named first. After the offset of the presentation of the second stimulus, the subject is required to name the stimuli S1 and S2 as quickly as possible.

The speech recording is automatically started at onset of the first stimulus, and the pause time duration from the end of presenting the second stimulus S2 to the first vocal pronunciation (articulation) of the first stimulus by the subject, is analyzed (Fig.3 in PCT/SE2010/050480)

The measured individual pause time (34) is here defined as the sum of the stimulus recognition time ts (lowest level in milliseconds, obtained in step 1), and the memory retrieval time tr_mean (The mean pause time for the 4 stimuli in step 2.) The sequence may be repeated, and for every repetition of the sequence of stimuli, an individual pause time is measured.

The method automatically subtracts the stimulus presentation times and the interstimulus interval from the recorded sound signal. As sound recording is started in the embodiment before the end of presentation of stimulus S2, any recording delays or measurement errors are avoided. The amount of contribution that the memory retrieval time has contributed to the individual pause time durations, is analyzed. Further, the recognition time is analyzed. Each individual pause time duration may be longer than the added recognition time and retrieval time, due to other brain activity components.

Thus, the signal provides a measure of the individual pause time durations, and components thereof.

The apparatus 4 is shown in an embodiment in Figure 4 in PCT/SE2010/05048 . All above described three steps 1-3 may be performed by means of the apparatus 4.

The apparatus 4 has a screen 42, a button 44 for adjusting the presentation time of the stimuli, a microphone 46, a start button 48, and a time interval selector section or scale 45; e.g. 1 = 0.1 seconds, 2 = 0.2 seconds etc. The apparatus 4 is preferably a portable hand held apparatus. Optionally the apparatus may comprise communication means to communicate diagnostic results obtained in a suitable context. The buttons 44, 46, 48, 49 may be so-called soft-buttons, i.e. visually presented buttons on a touch sensitive display.

Step 1 is performed by adjusting the stimulus recognition time by means of scale 45, e.g. showing discrete intervals, for instance ranging between 0.1 to 1.0 seconds. The adjustment is performed by button 44 whereby the presentation time ts of the stimulus can be adjusted upwards (+) and downwards (-). When the minimum presentation time for a correct recognition and naming of the stimulus is identified, the OK-button 49 is pressed by the examiner/subject and this stimulus presentation time ts is thereby set as default value by the apparatus.

Step 2 (Retrieval time probing working memory) is performed in the manner presented above. The sound is registered by means of the microphone 46 and recorded in a memory of the apparatus 4 for further processing.

After Steps 1 and 2 have been performed, the subject may press the START-button 48. At this occasion, pair wise stimuli may be presented on the screen according to Step 3. Two stimuli S1, S2 are shown on the screen 42 and turned on and off in successive order, separated by the presentation pause time, e.g. 0.1 sec as the default value. The duration of each stimulus presentation ts is defined in step 1.

The onset of sound recording starts at the onset of the first stimulus S1 and stops after the pronunciation of the second stimulus has occurred. Thereafter, a pause is made, e.g. 1 second elapses (can be adjusted if necessary), until the next presentation of a stimulus pair occurs automatically or is started manually. That means, once the START-button 48 is pressed, the test may continue automatically with an adjustable time interval between presentations, until a plurality, e.g. fourty, randomly generated pairs of stimuli S1, S2 have been presented and a plurality of individual pause times is measured for each pairs of stimuli. Thus, the presentation sequences runs automatically a pre-defined number of times, e.g. 40 times as default. The apparatus automatically stops presentation of stimuli according to step 3, when this number is reached.

The individual sequences may also be presented separately and each separate sequence is thereby started by the examiner/subject by holding down the START-button, which automatically activates the individual sequence.

The control unit is adapted to determine a statistical distribution of the plurality of individual pause times, and determine the occurrence and/or stage of brain disease induced brain dysfunction from the statistical distribution.

The statistical distribution of the individual pause times may be a chronological distribution. The distribution may be imposed on a range of normal reference or threshold values for a diagnosis. A threshold value may be defined as a number of individual pause times exceeding a threshold duration for a pause time. Non-overlapping pause times of the statistical distribution compared to a range of reference values may be detected for immediate indications of abnormal deviations in a temporally resolved manner.

### Further examples

Further examples, applications and uses in which the present invention may be beneficial are described below.

To assess any training effects on pause time duration, performed by a subject, either by physical training and exercise to improve brain blood flow and brain oxygenation and/or by any mental training programmes which are aimed to improve any cognitive abilities, such as for example memory function and reading and writing abilities, of that subject.

To assess the effects on pause time duration of any nutritional supplementations used by the subject, which supplementation is aimed to improve the physical and/or mental well-being of that subject. Such supplementations may involve any vitamin supplementation and any supplementation of any polyunsaturated fatty acids aimed to improve the lipid metabolism of the brain of that subject.

To assess the effects on pause time duration of any pharmaceutical intervention approach aimed at improving the transmission of any neurotransmitter subservient to any mental processes performed by the brain, such as for example any pharmaceutical drug present or developed in the future for the treatment of neurodevelopmental disorders such as ADHD, autism spectrum disorders or dementia disorders. Furthermore, to assess the effect on pause time duration by reducing the build-up of toxic by-products within the brain and/or to increase the elimination of toxic waste products of metabolism in the brain, via the blood-brain barrier and/or via the blood-cerebrospinal fluid barriers of the brain.

To assess the effects on pause time duration of any pharmaceutical and/or genetic intervention approach aimed at influencing or manipulating the cleavage processes by protease inhibitors of the amyloid precursor protein (APP), the protein which is thought to contribute to the build-up and the formation of neurofibrillary tangles and the formation of senile plaques (soluble or insoluble) within the brain parenchyma and the endothelial cells of the blood vessels in the brain, as these processes are thought to be at the core of the cognitive dysfunctions in Alzheimer's disease and vascular dementia.

To assess any effects on pause time duration of any pharmaceutical or genetic approach aimed to improve the symptoms of Parkinson's disease and Parkinson's dementia which affect any neurotransmitter system in the brain which overlaps with those neurotransmitter systems known to degenerate in Alzheimer's disease, dementia with Lewy bodies (also called Lewy body dementia), and Frontotemporal dementia.

To assess the effects on pause time duration of any other disorder than those mentioned earlier, which is known to slow down the brain's ability to process information, such as motorneuron disease, tumor, or stroke.

To assess the effect on pause time duration of any metabolic or otherwise dysfunction in other bodily organs than the brain of a subject, which can affect the cognitive performance of the brain.

The present invention has been described above with reference to specific embodiments. However, other embodiments than the above described are equally possible within the scope of the invention. Different method steps than those described above, performing the method by hardware or software, may be provided within the scope of the invention. The different features and steps of the invention may be combined in other combinations than those described. The scope of the invention is only limited by the appended patent claims.

## Claims

1. A diagnostic aid system devised for assessment of a brain status of a subject, wherein said brain status comprises a neurodevelopmental disorder or a brain disease induced brain dysfunction, wherein said system is adapted to determine an occurrence and/or stage of said neurodevelopmental order or brain disease induced brain dysfunction in said subject, said system comprising
an apparatus that is adapted to determine said occurrence and/or stage of said neurodevelopmental disorder or brain disease induced brain dysfunction in said subject from speech of said subject, said apparatus comprising units that are operatively connected to each other, comprising
a unit for registering said speech of said subject over a period of time;
a processing unit devised for analyzing said registered speech and configured to determine a plurality of individual pause time components of said registered speech and determine a statistical distribution of said plurality of individual pause time components; and
wherein said processing unit is adapted to determine said occurrence and/or stage of said brain disease induced brain dysfunction from said statistical distribution.

2. The system according to claim 1, wherein said processing unit that is adapted to determine occurrence and/or stage of said neurodevelopmental disorder or brain disease induced brain dysfunction is further adapted to compare said plurality of individual pause time components of said registered speech with pause time component threshold values to determine said occurrence and/or stage of said neurodevelopmental disorder or brain disease induced brain dysfunction.

3. The system according to claim 2, wherein said pause time component threshold values comprise a predefined number of said plurality of individual pause times exceeding a pause time threshold duration.

4. The system according to claim 3, wherein said pause time threshold duration is a predefined threshold duration outside of +/- one standard deviation of a reference pause time of a healthy subject.

5. The system according to claim 4, wherein said reference pause time is a range of reference pause times of a healthy population, and wherein said unit that is adapted to determine occurrence and/or stage of said neurodevelopmental disorder or brain disease induced brain dysfunction is adapted to determine overlapping values of pause times in said range with said statistical distribution of individual pause times for determining said occurrence and/or stage of said neurodevelopmental disorder or brain disease induced brain dysfunction.

6. The system according to any of the preceding claims wherein said statistical distribution is a chronological distribution of said plurality of individual pause time components.

7. The system according to any of the preceding claims, wherein said processing unit that is adapted to determine said occurrence and/or stage of said neurodevelopmental disorder or brain disease induced brain dysfunction from said statistical distribution is configured to not register naming errors.

8. The system according to any of claims 1 to 7, wherein said processing unit that is adapted to determine said occurrence and/or stage of said brain disease induced brain dysfunction from said statistical distribution is configured to associate a slowing of speech compartments with white matter function/dysfunction and/or cerebrovascular dysfunction, in either healthy aging, mild cognitive impairment (MCI) or dementia.

9. The system according to any preceding claim 1-8, wherein said assessment is cognitive test based assessment, wherein the subject is free to define parameters of said cognitive test, wherein said cognitive test is devised to provide measures of processing speed, such as for example using simple colors and shapes, or naming other defined stimuli, is non-invasive.

10. The system according to claim 9, wherein said cognitive test comprises presenting stimuli to said subject, wherein said stimuli comprises a sequence of individual visual and/or auditive stimuli in time for naming by said subject; or
wherein said cognitive test is implemented in an education and culture-free manner, and wherein said cognitive test does not comprise questions related to knowledge of said subject.

11. The system according to any preceding claim, wherein said unit for registering said speech comprises a unit for recording said speech of said subject over a period of time; and wherein said processing unit devised for analyzing said registered speech is a unit devised for analyzing said recorded speech and configured to determine said plurality of individual pause components of said speech, and/or wherein said apparatus is a handheld apparatus, and/or wherein said processing unit devised for analyzing is further devised for analyzing said speech of said subject irrespective of a language of said speech.

12. The system according to any preceding claim 1-11,
wherein said brain disease induced brain dysfunction is related to dementia, such as Alzheimer's disease; Multiple sclerosis (MS); an subcortical white matter disease or demyelization disease; HIV; malaria; cerebrovascular disease (VaD); encephalitis; traumatic brain injury (TBI); or mild cognitive impairment (MCI); traumatic brain injury (TBI); effects of street drugs; alcohol abuse; or side effects of prescribed drugs; pharmaceutical drug treatments, such as CNS, heart, or lung.

13. A computer program for processing by a computer, preferably said processing unit of said system of claims 1-12 or 14, storable on a computer-readable medium, wherein said computer program is configured for assessment of a brain status of a subject, wherein said brain status comprises a neurodevelopmental disorder or a brain disease induced brain dysfunction, wherein said computer program comprises
a first code segment for analyzing speech of said subject and determining a plurality of individual pause time components of said speech;
a second code segment for determining statistical distribution of said plurality of individual pause time components; and
a third code segment for determining an occurrence and/or stage of said neurodevelopmental disorder or a brain disease induced brain dysfunction in said subject based on said statistical distribution.

14. The system according to claim 1 to 12, wherein said neurodevelopmental disorder is related to ADHD, autism spectrum disorders (autism, Asperger's syndrome, PDD-NOS), global learning deficit or mental retardation, or comorbid neurological disease such as tumour and epilepsy, or a brain disease induced brain dysfunction related to dementia, such as Alzheimer's disease; Multiple sclerosis (MS); an subcortical white matter disease or demyelization disease; HIV; malaria; cerebrovascular disease (VaD); encephalitis; traumatic brain injury (TBI); or mild cognitive impairment (MCI); traumatic brain injury (TBI); effects of street drugs; alcohol abuse; or side effects of prescribed drugs; pharmaceutical drug treatments, such as CNS, heart, or lung; and/or wherein the duration of said individual pause times are affected by training performed by a subject such that said system is adapted to assess training effects on said individual pause times, wherein said training is either physical training and exercise to improve brain blood flow and brain oxygenation and/or by any mental training programmes which are aimed to improve any cognitive abilities, such as for example memory function and reading and writing abilities, of that subject,and/or wherein said individual pause time durations are affected by any nutritional supplementations used by the subject such that said system is adapted to assess effects of nutritional supplementations on said individual pause times, said supplementation is aimed to improve the physical and/or mental well-being of that subject, such supplementations comprising vitamin supplementation or supplementation of polyunsaturated fatty acids aimed to improve the lipid metabolism of the brain of the subject, and/or assessing the effects on said individual pause time durations of any pharmaceutical intervention approach aimed at improving the transmission of any neurotransmitter subservient to any mental processes performed by the brain, such as for example any pharmaceutical drug for the treatment of neurodevelopmental disorders (ADHD, autism spectrum disorders) or dementia disorders, and/or assessing the effect on said individual pause time durations by reducing the build-up of toxic by-products within the brain and/or to increase the elimination of toxic waste products of metabolism in the brain, via the blood-brain barrier and/or via the blood-cerebrospinal fluid barriers of the brain, and/or assessing the effects on said individual pause time durations of any pharmaceutical and/or genetic intervention approach aimed at influencing or manipulating the cleavage processes by protease inhibitors of the amyloid precursor protein (APP), and/or assessing effects on said individual pause time durations of pharmaceutical or genetic approach aimed to improve the symptoms of Parkinson's disease and Parkinson's dementia which affect any neurotransmitter system in the brain which overlaps with neurotransmitter systems that degenerate in Alzheimer's disease, dementia with Lewy bodies, and Frontotemporal dementia, and/or assessing the effects on said individual pause time durations of disorders that slow down the brain's ability to process information, such as tumor or stoke, and/or assessing the effect on said individual pause time durations of metabolic or other dysfunction in other bodily organs than the brain of the subject, which affect the cognitive performance of the brain.

15. A method for assessment of a brain status of a subject, wherein said assessment is performed internally in a system, preferably for performing by said system of claims 1-12 and 14 and/or by executing said computer program of claim 13, wherein said brain status comprises a neurodevelopmental disorder or a brain disease induced brain dysfunction, wherein said method comprises
analyzing speech of said subject and determining a plurality of individual pause components of said speech;
determining a statistical distribution of said plurality of individual pause time components; and
determining an occurrence and/or stage of said neurodevelopmental disorder or brain disease induced brain dysfunction in said subject based on said statistical distribution.
